# EUROPÄISCHE PATENTANMELDUNG

(11) **EP 1 092 386 A1**
(43) Veröffentlichungstag der Anmeldung: **18.04.2001**
(21) Anmeldenummer: 99810933.4
(22) Anmeldetag: 13.10.1999
(51) Int. Cl.: A61B 5/00, A61B 5/05

(54) **Verfahren und Vorrichtung zur Bestimmung einer Stoffkonzentration im Blut**

(71) Anmelder: Süsstrunk Anita, 8041 Zürich (CH)
(72) Erfinder: Süsstrunk, Heinz, 8041 Zürich (CH)
(74) Vertreter: Blum, Rudolf Emil Ernst

(57) **Zusammenfassung**

Zur unblutigen Bestimmung einer Stoffkonzentration im Blut, insbesondere zur Bestimmung des Blutzuckers, wird eine am Körper angeordnete elektrische Spule (L) periodisch von Strompulsen durchflossen. Nach den Strompulsen wird die Spannung (U_{L}) über der Spule ermittelt. Es zeigt sich, dass der Verlauf dieser Spannung (U_{L}) abhängig von der Konzentration zu messender Stoffe ist, falls die Frequenz der Strompulse einer heuristisch bestimmten Resonanzfrequenz entspricht. Des Messverfahren kann z.B. von einem Gerät in Form einer Armbanduhr durchgeführt werden.

## Beschreibung

Die Erfindung betrifft ein Verfahren zur unblutigen Bestimmung einer Stoffkonzentration im Blut und eine Vorrichtung zur Ausführung dieses Verfahrens gemäss Oberbegriff der unabhängigen Ansprüche. Solche Verfahren bzw. Vorrichtungen werden insbesondere zur Messung des Blutzuckergehaltes eingesetzt.

Zur Messung des Blutzuckergehaltes ist normalerweise eine Blutentnahme nötig. Da eine Blutentnahme aus offensichtlichen Gründen unerwünscht ist, werden alternative, "unblutige" Verfahren gesucht. Beispielsweise wurde versucht, den Blutzuckergehalt mittels Laserlicht zu bestimmen, was jedoch nicht sehr gut reproduzierbare Ergebnisse gibt, da die Resultate insbesondere stark von Temperatur, physischer Anstrengung, Sonnenbestrahlung etc. abhängen. Dies ist eine Konsequenz davon, dass bei der Messung mittels Laserlicht nur ein relativ kleiner subkutaner Bereich des Gewebes von ca. 3 mm Tiefe erfasst werden kann.

Es sind auch Vorrichtungen und Verfahren bekannt, bei denen der Blutzuckergehalt mittels Kernresonanz gemessen wird. Dies bedingt jedoch, dass sehr starke permanente Magnetfelder erzeugt werden, was entsprechende Apparaturen schwer und teuer macht.

Es stellt sich deshalb die Aufgabe, ein Verfahren und eine Vorrichtung der eingangs genannten Art bereitzustellen, die in einfacher Weise ohne Blutentnahme möglichst genaue Resultate liefern.

Diese Aufgabe wird von den unabhängigen Ansprüchen gelöst.

Erfindungsgemäss werden also intramolekulare Schwingungen in den Molekülen des zu bestimmenden Stoffs angeregt. Unter intramolekularen Schwingungen sind Schwingungen zu verstehen, die die Atome eines Moleküls gegeneinander ausführen. Ein von der Amplitude dieser Schwingungen abhängiges Messsignal wird sodann verwendet, um die Konzentration des Stoffs zu ermitteln.

Vorzugsweise werden die Moleküle mit einem elektromagnetischen Wechselfeld angeregt, welches insbesondere in einer Spule erzeugt wird, die an die Körperoberfläche gebracht wird. Es ist jedoch auch denkbar, die Schwingungen mechanisch mittels Schall- bzw. Ultraschallgeber zu erzeugen.

Das Messsignal kann zur Erhöhung der Genauigkeit mit einer optischen Reflexionsmessung kombiniert werden. Es zeigt sich, dass auf diese Weise die Abhängigkeit der Messung von der Blutmenge im Gewebe reduziert werden kann.

In einer bevorzugten Ausführung des Verfahrens wird eine elektromagnetische Spule in den Bereich der Körperoberfläche gebracht. Bei mindestens einer Frequenz wird sodann ein von der Induktivität der Spule abhängiger Messwert ermittelt und aus diesem wird, z.B. über eine geeignete Eichfunktion, die gesuchte Stoffkonzentration bestimmt.

Die erfindungsgemässe Vorrichtung besitzt vorzugsweise eine elektrische Spule, welche ein periodisch sich änderndes Magnetfeld erzeugt, und Messmittel, zur Ermittlung eines Messsignals aus dem Verlauf des Stroms bzw. der Spannung über der Spule. Im Gegensatz zu Messgeräten, die auf der Ermittlung kernresonanter Schwingungen basieren, ist jedoch keine Quelle zur Erzeugung eines permanenten Magnetfeldes von einer Grösse und Richtung vorgesehen, bei welchen bei der Anregungsfrequenz der Spule kernresonante Schwingungen auftreten könnten.

Das Verfahren bzw. die Vorrichtung eignen sich insbesondere zur Messung des Blutzuckers. In diesem Falle wird vorzugsweise eine Resonanz bei ca. 75.8 MHz angeregt.

Weitere Vorteile, bevorzugte Ausführungen und Anwendungen des Verfahrens bzw. der Vorrichtung ergeben sich aus den abhängigen Ansprüchen sowie aus der nun folgenden Beschreibung anhand der Figuren. Dabei zeigen:
Fig. 1 einen Schnitt durch eine Ausführung der erfindungsgemässen Vorrichtung,
Fig. 2 ein Blockschaltbild der Vorrichtung nach Fig. 1,
Fig. 3 die Ansteuerung der Messspule,
Fig. 4 der Verlauf der Spannungen in Fig. 3,
Fig. 5 eine vergleichende Tabelle von Mess-und Testresultaten.

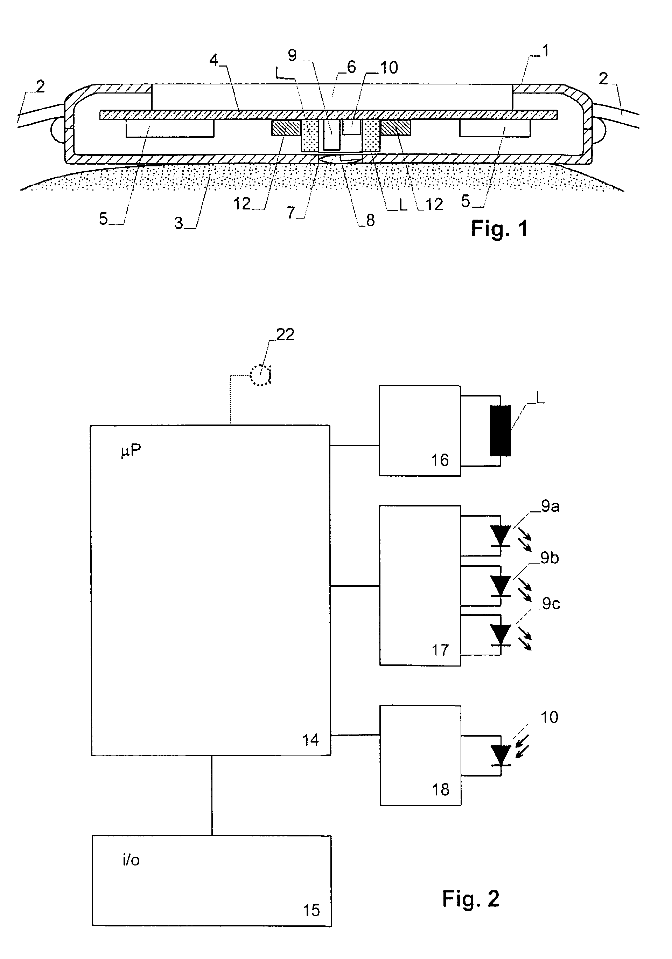

Ein bevorzugter mechanischer Aufbau der Vorrichtung in Form einer Armbanduhr ist in Fig. 1 dargestellt. Sie besitzt ein Gehäuse 1, welches von einem Armband 2 an der Körperoberfläche 3 gehalten wird. Im Gehäuse 1 ist ein Träger 4 angeordnet, welcher eine elektronische Schaltung 5 und eine Flüssigkristallanzeige 6 trägt. Am körperseitigen Boden des Gehäuses 1 ist eine Öffnung 7 ausgespart, in welcher eine Optik 8 angeordnet ist. Hinter der Optik ist ein Lichtquelle 9 und ein Lichtsensor 10 angeordnet, wobei der Lichtsensor 10 so positioniert ist, dass er vom Körper reflektiertes Licht der Lichtquelle 9 empfängt. Um die Lichtquelle 9 und den Lichtsensor 10 ist eine zylindrische elektrische Spule L angeordnet, deren Achse senkrecht zur Körperoberfläche steht. In oder neben der Spule L kann zusätzlich ein kleiner Permanentmagnet 12 angeordnet sein, dessen Feld im wesentlichen parallel zu jenem der Spule ist. Obwohl ein derartiger Permanentmagnet nicht unbedingt notwendig ist, so zeigt es sich, dass sein Feld die Qualität der Messsignale verbessert.

Fig. 2 zeigt ein Blockdiagramm der Schaltung des Geräts nach Fig. 1. Es besitzt einen Mikroprozessor 14, der mit einem Ein- und Ausgabeteil 15 verbunden ist. Letzterer Umfasst die Anzeige 6 sowie vom Benutzer betätigbare Bedienelemente konventioneller Art. Der Mikroprozessor 14 und der Ein- und Ausgabeteil 15 besitzen alle Funktionen einer normalen Armbanduhr. Darüber hinaus ist der Mikroprozessor jedoch in der Lage, den Blutzucker oder andere Stoffe im Körpergewebe zu ermitteln. Hierzu ist er über eine Ansteuerungsschaltung 16 mit der Spule L verbunden. Ferner ist eine Treiberstufe 17 vorgesehen, mit der die Lichtquelle 9, bestehend aus drei LEDs 9a, 9b, 9c unterschiedlicher Farbe (vorzugsweise Rot, Gelb und Grün oder Blau), angesteuert werden kann. Die Signale des Lichtsensors 10 gelangen über einen Verstärker 18 mit A/D-Wandler ebenfalls zum Mikroprozessor 14.

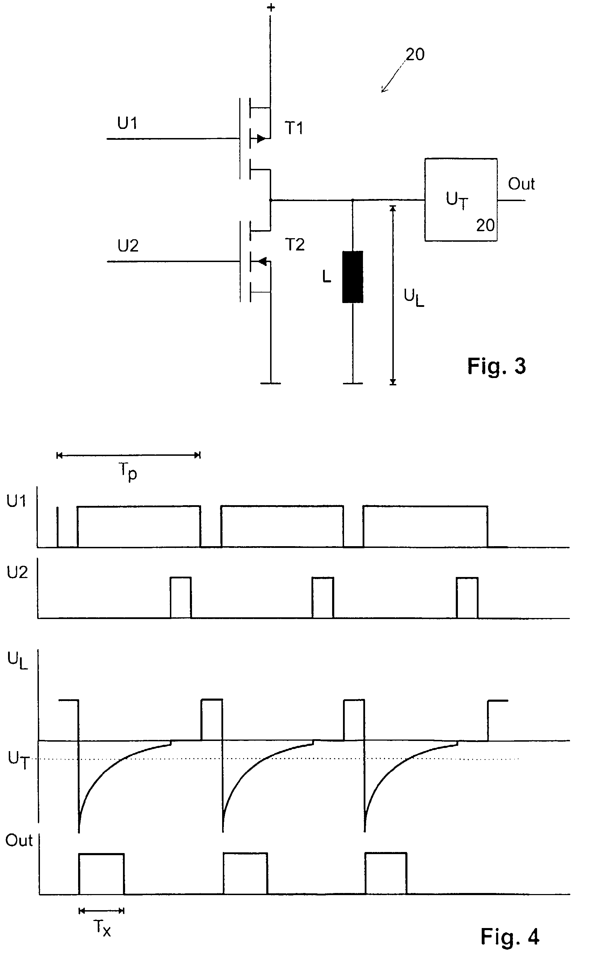

Die Ansteuerschaltung 16 für die Spule L ist in Fig. 3 dargestellt. Sie umfasst zwei komplementäre Transistoren T1, T2, die über Signale U1, U2 vom Mikroprozessor 14 einzeln gesteuert werden. Am Ausgang des Komplementärtransistor-Paars T1, T2, welches zwischen einer Versorgungsspannung und der Masse liegt, ist ein erster Anschluss der Spule L angeordnet. Der zweite Anschluss der Spule L liegt auf Masse. Ein Schwellwertdetektor 20 misst die Spannung U_{L} über der Spule und erzeugt ein Signal, sobald der Betrag der Spannung U_{L} über einem Schwellwert U_{T} liegt.

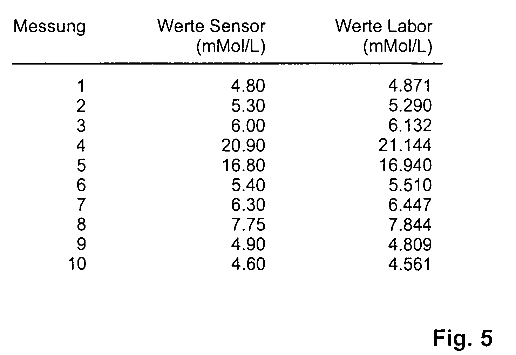

Der Betrieb der Ansteuerschaltung 16 ist in Fig. 4 illustriert. Der Mikroprozessor 15 schaltet zuerst den oberen Transistor T1 ein, wodurch die Spannung U_{L} über der Spule auf den Wert der positiven Versorgungsspannung ansteigt. Sodann wird Transistor T1 ausgeschaltet, was eine negative Induktionsspannung über der Spule erzeugt. Gleichzeitig geht der Ausgang "Out" des Schwellwertdetektors 20 von 0 auf 1. Wenn der Betrag der Spannung U_{L} nach einer Zeit T_{X} unter den Schwellwert U_{T} fällt, so geht der Ausgang "Out" von 1 auf 0. Sodann wird, nach einer vorgegebenen Zeit, der untere Transistor T2 eingeschaltet, um die Spannung über der Spule ganz zu entladen. Danach beginnt der Messzyklus von neuem.

Der Ausgang "Out" wird dem Mikroprozessor 15 zugeführt, welcher die Zeit Tₓ bestimmt. Diese Zeit Tₓ ist ein Mass für die Induktivität der Spule L, welche unter anderem auch von den magnetischen Eigenschaften des Gewebes bzw. des Bluts des Benutzers abhängt. Insbesondere zeigt es sich, dass die Spuleninduktivität bzw. der Wert von Tₓ eine Funktion der Blutzusammensetzung ist. Je nach Länge der Messperiode Tₚ bzw. der Anregungsfrequenz F = 1/Tₚ können dabei verschiedene Stoffe selektiv ausgemessen werden. So beträgt die bevorzugte Frequenz F zur Bestimmung des Blutzuckergehalts ca. 75.80 MHz, d.h. bei dieser Frequenz hängt der Wert der Spuleninduktivität bzw. der Zeit Tₓ stark vom Blutzuckergehalt ab.

Für andere Stoffe können andere Messfrequenzen gewählt werden, z.B. 75.95 MHz für die Bestimmung der Konzentration von NaCl in Lösung oder 86.4 MHz für Insulin. Die Messfrequenz für einen Stoff wird durch Messreihen bestimmt, wobei Proben unterschiedlicher Konzentration des Stoffs ausgemessen werden. Für jede Probe wird die Induktivität bzw. der Wert von Tₓ als Funktion der Frequenz F bestimmt. Sie so ermittelten Spektren werden miteinander verglichen, und es wird diejenige Frequenz als Messfrequenz bestimmt, bei der die stärkste Abhängigkeit des Messsignals von der Stoffkonzentration festgestellt wird. Ein bevorzugter Bereich der Frequenzen F liegt zwischen 10 MHz und 1 GHz, insbesondere zwischen 50 MHz und 200 MHz. Es sind jedoch auch Messungen bei anderen Frequenzen möglich.

In der vorliegenden Ausführung ermittelt das Gerät nur den Blutzuckergehalt und ist fest auf die Frequenz 75.87 MHz eingestellt. Es ist jedoch auch denkbar, dass die Messfrequenz variiert wird, um die Konzentration anderer Stoffe zu messen.

Der Wert des Messsignals hängt nicht nur von der Konzentration des zu messenden Stoffs, sondern auch von der Menge Blut im Messbereich ab. Da die Blutmenge z.B. aufgrund unterschiedlicher Durchblutung der Gefässe oder wegen Blutdruckschwankungen variieren kann, wird vorzugsweise eine zweite Messung durchgeführt. Diese zweite Messung kann entweder auch auf dem oben beschriebenen Verfahren basieren und z.B. die Konzentration eines zweiten Blutbestandteils im Messbereich bestimmen, wodurch auf die Menge des Bluts geschlossen und der Blutzuckerwert korrigiert werden kann.

Bevorzugt wird jedoch eine zusätzliche optische Messung durchgeführt. Hierzu wird die Grösse des vom Lichtsensor 10 empfangenen Signals, d.h. des reflektierten Lichts, ermittelt. Dieses Signal, d.h. der Reflektionskoeffizient des Körpers, hängt ebenfalls von der Blutmenge im ausgemessenen Gewebe ab.

Es zeigt sich, dass das Signal vom Lichtsensor 10, gegebenenfalls nach geeigneter Skalierung, einfach zum Wert T_{X} addiert werden kann, um verlässlichere Resultate zu erhalten.

Vorzugsweise wird das Messsignal T_{X}, gegebenenfalls nach einer Addition mit dem Signal vom Lichtsensor, über eine Eichtabelle oder einen Eichfaktor in die gesuchte Blutzuckerkonzentration umgewandelt. Hierzu wird in einem Kalibrierschritt das Messsignal mit einem Blutzuckerwert verglichen, der in konventioneller Art ermittelt wurde. Hieraus kann eine Eichfunktion (bestehend z.B. aus einem Eichfaktor oder einer Eichtabelle) ermittelt werden. Vorzugsweise wird dieser Eichschritt für jeden Anwender neu durchgeführt.

Im hier gezeigten Ausführungsbeispiel wird mittels dreier Leuchtdioden unterschiedlicher Farbe Licht mit sehr breitem Spektrum erzeugt. Es ist auch denkbar, andere Lichtquellen einzusetzen.

Fig. 5 zeigt eine Tabelle von Messungen einer geeichten Vorrichtung im Vergleich mit analytisch gewonnenen Referenzresultaten. Es zeigt sich, dass das vorliegende Verfahren eine hohe Genauigkeit besitzt.

Die Erfinder gehen davon aus, dass im vorliegenden Verfahren durch die Magnetpulse der Spule L intramolekulare Schwingungen in den Molekülen des gesuchten Stoffs angeregt werden, und dies insbesondere auch in Frequenzbereichen unterhalb 1 GHz. Entspricht die Messfrequenz einer Resonanz im Molekül, so ist das entsprechende Messsignal besonders stark. Die Induktivität der Spule L bzw. der Wert der Zeit T_{X} ist dabei abhängig von der Amplitude der angeregten Schwingungen.

Bevorzugte Messbereiche sind 10 MHz bis 1 GHz, wobei sich in der Praxis ein Bereich zwischen 50 MHz und 200 MHz als besonders günstig herausgestellt hat.

Im vorliegenden Fall wird zur Anregung ein periodisches elektromagnetisches Signal verwendet und eine Kopplung des elektromagnetischen Feldes mit den Atomen und/oder Bindungen des Moleküls ausgenützt. Es wird jedoch auch vorgeschlagen, anstelle der (oder zusätzlich zur) Spule L einen piezoelektrischen Schall- oder Ultraschallgeber 22 einzusetzen, der mechanische Schwingungen erzeugt und entsprechende Echos auffängt.

## Patentansprüche

1. Verfahren zur unblutigen Bestimmung einer Stoffkonzentration im Blut, insbesondere zur Bestimmung des Blutzuckers, dadurch gekennzeichnet, dass die Moleküle des zu bestimmenden Stoffs auf einer Resonanzfrequenz zu intramolekularen Schwingungen angeregt werden und dass ein von der Amplitude der Schwingungen abhängiges Messsignal (T_{X}) gemessen wird.

2. Verfahren nach Anspruch 1, dadurch gekennzeichnet, dass die Schwingungen mittels eines periodischen elektromagnetischen Signals angeregt werden.

3. Verfahren nach Anspruch 2, dadurch gekennzeichnet, dass das elektromagnetische Signal in einer an eine Körperoberfläche gebrachten Spule (L) erzeugt wird.

4. Verfahren nach Anspruch 3, dadurch gekennzeichnet, dass die Spule (L) mit periodisch variierendem Strom beaufschlagt wird, wobei aus dem Verlauf einer an der Spule abfallenden elektrischen Spannung (U_{L}) die Stoffkonzentration ermittelt wird.

5. Verfahren nach Anspruch 4, dadurch gekennzeichnet, dass an die Spule (L) mindestens ein Spannungspuls angelegt wird, wobei nach dem Puls der Abfall der über der Spule (L) liegenden Spannung (U_{L}) ermittelt wird, und insbesondere dass bestimmt wird, wann die Spannung (U_{L}) unter einen vorgegebenen Wert (U_{T}) sinkt.

6. Verfahren nach einem der Ansprüche 2 bis 5, dadurch gekennzeichnet, dass die Induktivität (L) der Spule (L) bei der Resonanzfrequenz gemessen wird.

7. Verfahren nach Anspruch 1, dadurch gekennzeichnet, dass die Schwingungen mittels eines Schall-bzw. Ultraschallgebers (25) mechanisch angeregt werden.

8. Verfahren nach einem der vorangehenden Ansprüche, dadurch gekennzeichnet, dass die Schwingungen im Bereich zwischen 10 MHz und 1 GHz, insbesondere zwischen 50 MHz und 200 MHz, zur Messung des Blutzuckers bevorzugt bei ca. 75.8 MHz, angeregt werden.

9. Verfahren nach Anspruch 8, dadurch gekennzeichnet, dass die Frequenz der Anregung durchgestimmt wird, um die Konzentration verschiedener Stoffe zu bestimmen.

10. Verfahren nach einem der vorangehenden Ansprüche, dadurch gekennzeichnet, dass zusätzlich Licht in die Körperoberfläche eingestrahlt und die Menge des reflektierten Lichts gemessen wird, wobei aus der Menge des reflektierten Lichts und dem von der Amplitude der Schwingungen abhängigen Messsignal die Stoffkonzentration ermittelt wird.

11. Verfahren nach Anspruch 10, dadurch gekennzeichnet, dass ein aus der Menge des reflektierten Lichts ermittelter Reflektionskoeffizient zur Bestimmung der Stoffkonzentration additiv mit dem von der Amplitude der Schwingungen abhängigen Messsignal verknüpft wird.

12. Verfahren nach einem der vorangehenden Ansprüche, dadurch gekennzeichnet, dass das von der Amplitude der Schwingungen abhängige Messsignal in einem Kalibrierschritt mit in anderer Weise ermittelten Stoffkonzentrationen verglichen wird und dass hieraus eine Eichfunktion festgelegt wird, die das Messsignal mit der Stoffkonzentration verknüpft.

13. Verfahren zur unblutigen Bestimmung einer Stoffkonzentration im Blut, insbesondere nach einem der vorangehenden Ansprüche, dadurch gekennzeichnet, dass eine elektromagnetische Spule (L) in den Bereich einer Körperoberfläche gebracht wird, dass bei mindestens einer Frequenz ein von der Induktivität (L) der Spule abhängiger Messwert (T_{X}) gemessen wird, und dass aus dem Messwert (T_{X}) die Stoffkonzentration bestimmt wird.

14. Vorrichtung zur Bestimmung einer Stoffkonzentration im Blut, insbesondere zur Bestimmung des Blutzuckers, gekennzeichnet durch
eine elektrische Spule (L),
Befestigungsmittel (2) zum Anbringen der Spule im Bereich einer Körperoberfläche,
eine Treiberschaltung (T1, T2) zur Erzeugung eines periodischen Stroms in der Spule bei einer Frequenz (F) oder in einem Frequenzbereich und
Messmitteln (20) zur Ermittlung mindestens eines Messsignals (T_{X}) aus dem Verlauf des Stroms und/oder aus einer an der Spule abfallenden elektrischen Spannung,
wobei die Vorrichtung kein permanentes Magnetfeld von einer Grösse und Richtung erzeugt, bei welchen bei der Anregung durch die Spule (L) kernresonante Schwingungen auftreten könnten.

15. Vorrichtung nach Anspruch 14, dadurch gekennzeichnet, dass sie eine Lichtquelle (9) zum Einstrahlen von Licht auf die Körperoberfläche und einen Lichtsensor (10) zum Messen von reflektiertem Licht aufweist, sowie Mittel zur additiven Verknüpfung eines vom Lichtsensor gemessenen Reflektionsanteils des Lichts und des Messsignals (T_{X})

16. Vorrichtung nach Anspruch 15, dadurch gekennzeichnet, dass die Spule (L) um die Lichtquelle (9) und den Lichtsensor (10) herum angeordnet ist.

17. Vorrichtung nach einem der Ansprüche 14-16, dadurch gekennzeichnet, dass sie als Armbanduhr ausgestaltet ist.

18. Vorrichtung nach einem der Ansprüche 14-17, dadurch gekennzeichnet, dass sie eine Quelle (12) zur Erzeugung eines permanenten Magnetfeldes aufweist, und insbesondere dass das permanente Magnetfeld im wesentlichen parallel zu dem in der Spule erzeugten Magnetfeld ist.

19. Vorrichtung nach einem der Ansprüche 14 - 18, dadurch gekennzeichnet, dass der periodische Strom eine Grundfrequenz zwischen 10 MHz und 1 GHz, insbesondere zwischen 50 MHz und 200 MHz, zur Messung des Blutzuckers bevorzugt ca. 75.8 MHz, aufweist.
